# EUROPÄISCHE PATENTSCHRIFT

(11) **EP 1 686 956 B1**
(45) Veröffentlichungstag und Bekanntmachung des Hinweises auf die Patenterteilung: **24.07.2013**
(21) Anmeldenummer: 04818815.5
(22) Anmeldetag: 19.10.2004
(51) Int. Cl.: A61K 8/46, A61K 8/42, A61Q 5/02, A61Q 19/10

(54) **Verwendung von Tensidsystemen zur Verringerung der Schädigung hauteigener Enzyme**
Use of surfactant systems for reducing skin-intrinsic enzyme damage
Utilisation de systèmes de tensioactifs pour diminuer la déterioration des enzymes corporelles

(30) Priorität: 19.11.2003 DE 10354115
(43) Veröffentlichungstag der Anmeldung: 09.08.2006
(73) Patentinhaber: Beiersdorf AG, 20245 Hamburg (DE)
(72) Erfinder: SCHEPKY, Andreas, 25474 Bönningstedt (DE); RUPPERT, Stephan, 20259 Hamburg (DE); WILKEN, Maren, 22848 Norderstedt (DE); FRESE, Christian, 22765 Hamburg (DE); SIEGNER, Ralf, 25421 Pinneberg (DE); HOLTZMANN, Ursula, 22309 Hamburg (DE); KAUFFELDT, Martin, 22307 Hamburg (DE)
(86) Internationale Anmeldenummer: PCT/EP2004/052577
(87) Internationale Veröffentlichungsnummer: WO 2005/048971

(56) Entgegenhaltungen:
- EP-A- 0 556 660
- EP-A- 1 384 467
- EP-A- 1 393 714
- WO-A-01/85106
- WO-A-03/084486
- WO-A-2004/006870
- WO-A-2004/022012
- DE-A1- 19 529 773
- SCHEPKY A G ET AL: "INFLUENCE OF CLEANSING ON STRATUM CORNEUM TRYPTIC ENZYME IN HUMAN SKIN", INTERNATIONAL JOURNAL OF COSMETIC SCIENCE, KLUWER ACADEMIC PUBLISHERS, DORDRECHT, NL, vol. 26, no. 5, 1 January 2004 (2004-01-01), pages 245-253, XP009057577, ISSN: 0142-5463, DOI: 10.1111/J.1467-2494.2004.00232.X

## Beschreibung

Die vorliegende Erfindung betrifft die Verwendung von Tensidsystemen aus Laurylethersulfat, Alkylamidopropylbetain und einem weiteren Tensid zur Verringerung der Schädigung hauteigener Enzyme bei der Körper- oder Haarreinigung.

### Definitionen

Hauteigene Enzyme im Sinne der vorliegenden Schrift sind Enzyme, die auf der Hautoberfläche oder nahe der Oberfläche in der Haut vorliegen. Solche Enzyme können beispielsweise Hydrolasen, wie Proteasen, Esterasen, Lipasen, Phosphatasen, Sulfatasen und Transglutaminasen, insbesondere jedoch Proteasen wie das Stratum Corneum Tryptisches Enzym sein. In Tabelle 1 und 2 sind die wichtigsten aus der Literatur bekannten "Stratum Corneum Enzyme" aufgezeigt.

**Tabelle 1: Enzyme, die Desmosomen degradieren und zur Desquamation beitragen**

| **Enzym** | **Wirkort** | **Reaktion (Barriereschaden)** | **Literatur** |
|---|---|---|---|
| SCCE | SC (LB) | Spaltung von Proteinbindungen | Lundström, 1991 Suzuki, 1994 Sondell, 1995 Chang-Yi, 1997 |
| Trypsin | SC | Spaltung von Proteinbindungen | Suzuki, 1994 Chang-Yi, 1997 |
| Cathepsine | SG | Filaggrinabbau Keratinisierungshilfe | Hara, 1993 Kawada, 1997 |
| Thiol-Protease | SC | | Yokozeki, 1987 |

**Tabelle 2: Enzyme, die die Barriere aufbauen und zur Barrierehomöostase beitragen**

| **Enzym** | **Wirkort** | **Reaktion (Barriereschaden)** | **Literatur** |
|---|---|---|---|
| Phospholipase A₂ | SG-SC; LB | Freisetzung von Fettsäuren und möglicherweise Cholesterol von Cholesterolestern | Mauro, 1998 Mao-Qiang, 1995 Elias, 1988 Menon, 1986 |
| Saure Lipase | SC, LB | Freisetzung von Sterolen | Menon, 1986 Elias, 1988 |
| Neutrale Lipase | SC, LB | Sterol - und Fettsäurefreisetzung Regulation von Proteinkinasen (Different.) | Menon, 1986 |
| Sphingomyelinase | SC, LB | Bereitstellung von Ceramiden | Menon, 1986 |
| Ceramidase | SC | Keinen | Jin, 1994 |
| ß-Glucocerebrosidase | SC | Konversion von Glycoceramiden zu Ceramiden | Holleran, 1992 Mauro, 1998 |
| Steroid Sulfatase | SC | Cholesterolfreisetzung aus Cholesterolsulfat | Elias, 1988 |
| Sulfatasen | SC | Precursor-Spaltung | Baden, 1980 |

**Ammonia-Lyasen** spielen eine wichtige Rolle beim **Filaggrinabbau** (*Kuroda et al.,* 1979). Ebenso wie **Transglutaminasen** (*Polakowska et al.,* 1991), die für die Bildung des **'Cornified Envelope'** essentiell sind. **Phosphatasen** sind die Hydrolasen mit der höchsten Gesamtaktivität im Stratum Corneum.

Schädigung hauteigener Enzyme im Sinne der vorliegenden Schrift meint jede Form von Inaktivierung dieser Enzyme durch Denaturierung, Inhibierung oder chemischen Abbau. Kommen Enzyme mit Tensiden in Kontakt, so kommt es sehr häufig zu einer Denaturierung. *Prottey et al*., 1984 quantifizierten den Effekt von Tensiden auf die saure Phosphatase des Stratum Comeums (erhalten durch Tapestripping) durch Messung der Phosphatase-Aktivität. Hierbei konnte eine Reduktion der Enzymaktivität durch Denaturierung des Enzyms festgestellt werden. Aufgrund weiterer Daten ist von einer Tensidempfindlichkeit der meisten oberflächenaktiven Hautenzyme auszugehen.

Die bekannten Produkte zur Reinigung der Haut enthalten beispielsweise Mischungen aus Laurylethersulfat und Alkylamidopropylbetain. Durch Anwendung solcher Produkte kommt es zu einer teilweisen Denaturierung der hauteigenen Enzyme und somit zu einer Schädigung der Haut, da diese Enzyme physilogisch eine wichtige Rolle innehaben.

Die Schrift WO 2000/11124 offenbart waschaktive Zubereitungen mit mehr als 9% Laurylethersulfat und N-Acylaminosäuren. Diese lassen bei ihrer Anwendung besonders wenig Tensid auf der Hautoberfläche adhäsiv zurück und wirken so einer Schädigung der Haut durch eine Tensidbeladung entgegen. Erfindungsgemäße Tensidkombinationen werden nicht offenbart.

Die Schrift EP 1210933 offenbart Hautkonditioniermittel, die Amoniumsalze oder deren Ionen und R1R2R3C-CR4R5-NR6R7 enthalten, wobei R1, R2 and R3 jeweils H, OH, Niederalkyl, Phosphoryloxy, Aryl, R4 and R5 jeweils H, OH, Niederalkyl, Phosphoryloxy, Aryl, oder R4 and R5 gemeinsam m eine Carbonylgruppe bilden;
R6 and R7 jeweils H, Niederalkyl, oder aber R6 and R2 stellen Alkylengruppen dar, die zusammen mit dem sie tragenden C-Atom einen Fünfring bilden. Erfindungsgemäße Tensidkombinationen werden dagegen nicht offenbart.

WO-A-03/084486, DE-A-19529773, EP-A-556660 und WO-A-01/85106 beschreiben kosmetische oder dermatologische Reinigungszusammensetzungen, die Tensidmischungen aus Alkylethersulfat, Alkylamidopropylbetain und einem weiteren Tensid enthalten.

Es hat sich für den Fachmann nicht vorhersehbar herausgestellt, dass kosmetische und/oder dermatologische Körperreinigungszubereitung mit verringerter Neigung zur Schädigung hauteigener Enzyme enthaltend ein Tensidsystem aus (1) Alkylethersulfat, (2) Alkylamidopropylbetain, (3) einem weiteren Tensid gewählt aus der Gruppe Alkylpolyglucoside, Ethoxylierte Triglyceride und Salze von Citronensäurealkylpolyglycolester den Mängeln des Standes der Technik abhelfen. Durch solche Zubereitungen bzw. deren Anwendung wird eine verminderte Hautschuppigkeit, verminderte Hautrauigkeit, verbesserte Hautfeuchtigkeit, verbesserte Barriereintegrität und -funktion, eine verbesserte Hautintegrität, -elastizität, eine verbesserte Hautphysiologie sowie verbesserte Haaranhangsphysiologie im Vergleich zum Stand der Technik bewirkt.

Die Erfindung umfasst die Verwendung von Tensidsystemen aus (1) Alkylethersulfat, (2) Alkylamidopropylbetain, (3) einem weiteren Tensid gewählt aus der Gruppe Alkylpolyglucoside, Ethoxylierte Triglyceride und Salze von Citronensäurealkylpolyglycolester-sulfosuccinaten zur Verringerung der Schädigung hauteigener Enzyme bei der Körper- oder Haarreinigung.

Dabei ist es bevorzugt, wenn der Gehalt an weiterem Tensid (3) größer 1 Gew.%, bevorzugt größer 1,5 Gew.% ist. Weiter bevorzugt ist es, wenn das Verhältnis von Alkylethersulfat zu weiterem Tensid 10:0,5 bis 10:5, besonders bevorzugt 10:1 bis 10:3 und ganz besonders bevorzugt 10:1,8 bis 10:2,2 beträgt. Dadurch werden auch nach häufigem Duschen die hauterneuernden Enzyme geschützt und somit das natürliche Gleichgewicht der Haut unterstützt. Bevorzugt ist auch, wenn in den Zubereitungen zusätzlich Na-Cocoylglutamat enthalten ist. Besonders bevorzugt ist es, wenn als Tensid (3) PEG-7 Glycerylcocoat, Dinatrium PEG-5 Laurylcitrat Sulfosuccinat oder Laurylglucosid gewählt wird.

### Alkylpolyglucoside werden durch die Strukturformel

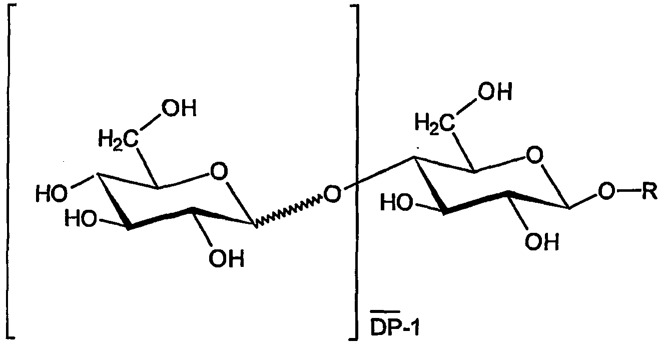

gekennzeichnet, wobei R einen verzweigten oder unverzweigten Alkylrest mit 4 bis 24 Kohlenstoffatomen darstellt und wobei *D̅P̅* einen mittleren Glucosylierungsgrad von bis zu 2 bedeutet.

Besonders bevorzugt sind Decylpolyglucosid bzw. Laurylpolyglucosid, die von der Firma Cognis unter den Handelsnamen Plantacare 2000 oder Plantaren 2000 bzw. Plantaren 1200 vertrieben werden.

Ethoxylierte Glycerin-Fettsäureester (Ethoxylierte Triglyceride) werden in wäßrigen Reinigungsrezepturen zu verschiedenen Zwecken eingesetzt. Niedrig ethoxylierte Glycerin-Fettsäureester (EO 3-12) dienen üblicherweise als Rückfetter zur Verbesserung des Hautgefühls nach dem Abtrocknen, Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt. Erfindungsgemäß vorteilhaft werden die ethoxylierten Triglyceride gewählt aus der Gruppe der ethoxylierten Glycerin-Fettsäureester, insbesondere bevorzugt: PEG-10 Olivenölglyceride, PEG-11 Avocadoölglyceride, PEG-11 Kakaobutterglyceride, PEG-13 Sonnenblumenölglyceride, PEG-15 Glycerylisostearat, PEG-9 Kokosfettsäureglyceride, PEG-54 Hydriertes Ricinusöl, PEG-7 Hydriertes Ricinusöl, PEG-60 Hydriertes Ricinusöl, Jojobaöl Ethoxylat (PEG-26 Jojoba-Fettsäuren, PEG-26 Jojobaalkohol), Glycereth-5 Cocoat, PEG-9 Kokosfettsäureglyceride, PEG-7 Glycerylcocoat, PEG-45 Palmkernölglyceride, PEG-35 Ricinusöl, Olivenöl-PEG-7 Ester, PEG-6 Caprylisäure/Caprinsäureglyceride, PEG-10 Olivenölglyceride, PEG-13 Sonnenblumenölglyceride, PEG-7 Hydriertes Ricinusöl, Hydrierte Palmkernölglycerid-PEG-6 Ester, PEG-20 Maisölglyceride, PEG-18 Glyceryloleat/-cocoat, PEG-40 Hydriertes Ricinusöl, PEG-40 Ricinusöl, PEG-60 Hydriertes Ricinusöl, PEG-60 Maisölglyceride, PEG-54 Hydriertes Ricinusöl, PEG-45 Palmkernölglyceride, PEG-35 Ricinusöl, PEG-80 Glycerylcocoat, PEG-60 Mandelölglyceride, PEG-60 "Evening Primrose" Glyceride, PEG-200 Hydriertes Glycerylpalmat, PEG-90 Glycerylisostearat.

Bevorzugte ethoxylierte Öle sind solche mit einem Ethoxylierungsgrad von 3-15, besonders bevorzugt PEG-7 Glycerylcocoat oder PEG-9 Kokosglyceride, die im Handel unter der Bezeichnung: Tegosoft GC von der Firma Goldschmidt, Cetiol HE von der Firma Cognis bzw. als Oxypon 401 von der Firma Zschimmer & Schwarz erhältlich sind. Ganz besonders bevorzugt sind ethoxylierte Triglyceride mit einem Gehalt an ein- oder mehrfach ethoxyliertem Glycerin von >20%.

Salze von Citronensäurealkylpolyglycolester-sulfosuccinaten 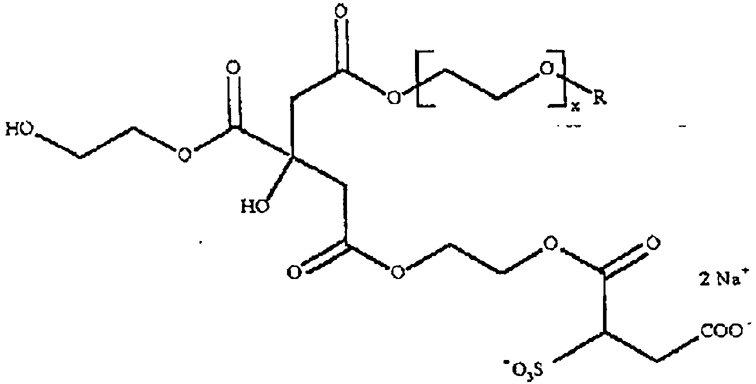 weisen bevorzugt einen Ethoxylierungsgrad x von 3-10, besonders bevorzugt einen mittleren Ethoxylierungsgrad von 5 auf. Bevorzugt ist R = Cocoyl, Palmitoyl oder Lauryl. Besonders bevorzugt sind Disodium PEG-5 Laurylcitrate Sulfosuccinate, dass von der Firma Goldschmidt unter dem Namen Rewopol SB CS 50 vertrieben wird.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erfordedich, um die Erfindung auszuführen.

Erfindungsgemäße Zubereitungen können weiterhin Tenside enthalten . Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und - lösung, ein leichtes Abspülen und - je nach Wunsch -für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻. während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine Ionen.

### A. Anionische Tenside

Vorteilhaft zu verwendende anionische Tenside sind Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,
Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.

Sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Aikyi- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkylamidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Vorteilhaft liegen Reinigungszubereitungen gemäß der Erfindung in Form von Gelen vor und enthalten einen oder mehrere Gelbildner bzw. Hydrokolloide.

"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wäßrigen Medien ist Voraussetzung dafür ist, daß diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

| | | | | |
|---|---|---|---|---|
| —NH₂ | —COOH | -COO⁻ | M⁺ | 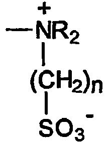 |
| —NH—R | 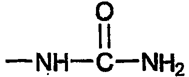 | -SO₃⁻ | M⁺ | |
| —OH | | -PO²⁻₃ | M²⁺ | |
| —SH | 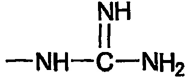 | + -NH₃ | X⁻ | 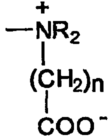 |
| —O— | | + —NR₂H | X⁻ | |
| 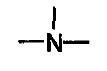 | 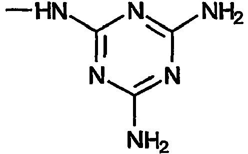 | + —NR₃ | X⁻ + | |
| | | —PR₃ | X⁻ | |
| | | 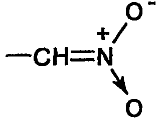 | | |

Die Gruppe der kosmetisch und dermatologisch relevanten Hydrokolloide läßt sich wie folgt einteilen in:
organische, natürliche Verbindungen, wie beispielsweise Agar-Agar, Carrageen, Tragant, Gummi arabicum, Alginate, Pektine, Polyosen, Guar-Mehl, Johannisbrotbaumkemmehl, Stärke, Dextrine, Gelatine, Casein,
organische, abgewandelte Naturstoffe, wie z. B. Carboxymethylcellulose und andere Celluloseether, Hydroxyethyl- und -propylcellulose und dergleichen,
organische, vollsynthetische Verbindungen, wie z. B. Polyacryl- und Polymethacryl-Verbindungen, Vinylpolymere, Polycarbonsäuren, Polyether, Polyimine, Polyamide,
anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus 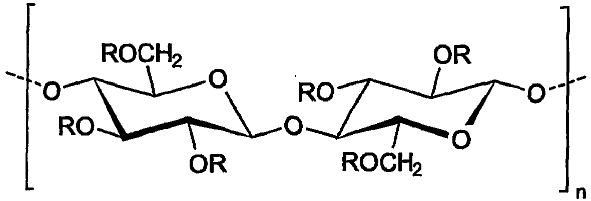 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH2-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2x106 bis 24x106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 106 produziert. Xanthan wird aus einer Kette mit β-1,4-gebundener Glucose (Cellulose) mit Seitenketten gebildet Die Struktur der Untergruppen besteht aus Glucose, Mannose, Glucuro nsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlant, zu den Florideen zählenden Rotalgen (Chondrus crispus u. Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht lösl. ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt. Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute τ-Carrageenan ist sowohl wasserlöslich als auch gelbildend. Weitere Carrageen-Typen werden ebenfalls mit griechischen Buchstaben bezeichnet α, β, γ, µ, v, ξ, π, ω, χ. Auch die Art vorhandener Kationen (K+, NH4+, Na+, Mg2+, Ca2+) beeinflußt die Löslichkeit der Carrageene.

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

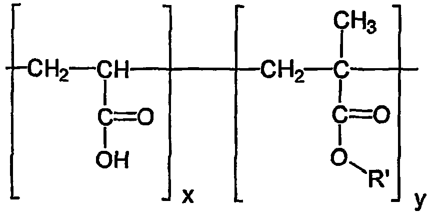

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der NOVEON Inc. erhältlichen.

Vorteilhaft sind ferner Verbindungen, die die INCI-Bezeichnung "acrylates/C12-24 pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der International Specialty Products Corp. erhältlich), die die INCI-Bezeichnung "acrylates/steareth-20 itaconate copolymer (unter der Handelsbezeichnungen Structure 2001® bei der National Starch erhältlich), die die INCI-Bezeichnung "acrylateslaminoacrylates/C10-30 alkyl PEG-20 itaconate copolymer" (unter der Handelsbezeichnungen Structure Plus® bei der National Starch erhältlich) und ähnliche Polymere.

Die Gesamtmenge an einem oder mehreren Hydrokolloiden wird in den fertigen kosmetischen oder dermatologischen Zubereitungen vorteilhaft kleiner als 1,5 Gew.-%, bevorzugt zwischen 0,1 und 1,0 Gew.-%, bezogen auf das Gesamtgewicht der Zubereitungen, gewählt.

Es ist vorteilhaft im Sinn der vorliegenden Erfindung, wenn der Gehalt an einem oder mehreren Polyacrylaten in der kosmetischen oder dermatologischen Reinigungsemulsion aus dem Bereich von 0,5 bis 4 Gew.-%, ganz besonders vorteilhaft von 0,7 bis 2 Gew.-% gewählt wird, jeweils bezogen auf das Gesamtgewicht der Zubereitungen.

Es ist auch von Vorteil, den erfindungsgemäßen Zubereitungen Komplexbildner zuzusetzen. Vorteilhaft werden die Komplexbildner gewählt aus der Gruppe bestehend aus Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen, Tetrasodium Iminodisuccinate, Trisodium Etylenediamine Disuccinate.

Weiterhin können in den kosmetischen Reinigungsmitteln Konditionierhilfsmittel enthalten sein, z.B. in Mengen von 0,001 bis 10 Gew.-%, bezogen auf das Gesamtgewicht der Zubereitungen.
Zu den bevorzugten Konditionierhilfsmittein gehören kationische Polymere, die für eine Verbesserung der Pflegeeigenschaften am Haar sorgen.
Dazu gehören kationische Cellulose-Derivate synthetisiert auf Basis von Hydroxycellulose mit einem trimethylammoniumsubstituierten Epoxid. Diese Substanzen sind unter der Bezeichnung Polyquaternium-10 bekannt und kommerziell z.B. als Polymer JR 400 von der Union Carbide Cooperation erhältlich.

Weitere Substanzen z.B.: kationische Polysaccharide besonders modifizierte Guarderivate, bekannt unter der Bezeichnung JAGUAR C13S und vertrieben durch Meyhall.; Homo- und Copolymere auf Basis von (Meth)acryloyloxyethyltrimethylammoniumsalz mit dem Handelnamen Salcare SC92 oder Salcare SC95 erhältlich bei Allied Colloids; Polymere auf Basis des Monomers Diallyldimethylammoniumchlorid wie Polyquaternium-6 als Homopolymer mit dem Handelsnamen Salcare SC30 und Polyquaternium-7 als Copolymer mit Acrylamid unter dem Handelsnamen Salcare SC10; Polyquatemium-47 als Copolymer von Acrylsäure,Methacrylat und Methacrylamidopropyltrimoniumchlorid mit dem Handelsnamen Merquat 2001 N von der Firma Calgon; Copolymere von Vinylpyrrolidone und Vinylmethylimidazolium Salz wie Polyquaternium-44 erhältlich als Luviquat Care von BASF; Terpolymere von Vinylpyrrolidon, Dimethylaminopropylmethacrylamid und Alkyldimethylaminopropylmethacrylamidoammoniumsalze unter dem Handelsnamen Styleze W-20 von der Firma ISP.

In der Lebensmitteltechnologie zugelassene Konservierungsmittel, die mit ihrer E-Nummer nachfolgend aufgeführt sind, sind erfindungsgemäß vorteilhaft zu verwenden.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner sind erfindungsgemäß in der Kosmetik gebräuchliche Konervierungsmittel oder Konservierungshilfsstoffe Dibromdicyanobutan (2-Brom-2-brommethylglutarodinitril), 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, DMDM Hydantoin , IPBC (Formaldehydabspalter) geeignet.

Ferner sind Phenylhydroxyalkylether, insbesondere die unter der Bezeichnung Phenoxyethanol bekannte Verbindung aufgrund ihrer bakteriziden und fungiziden Wirkungen auf eine Anzahl von Mikroorganismen als Konservierungsmittel geeignet

Auch andere keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Famesol (3,7,11-Trimethyl-2,6,10-dodecatrien-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

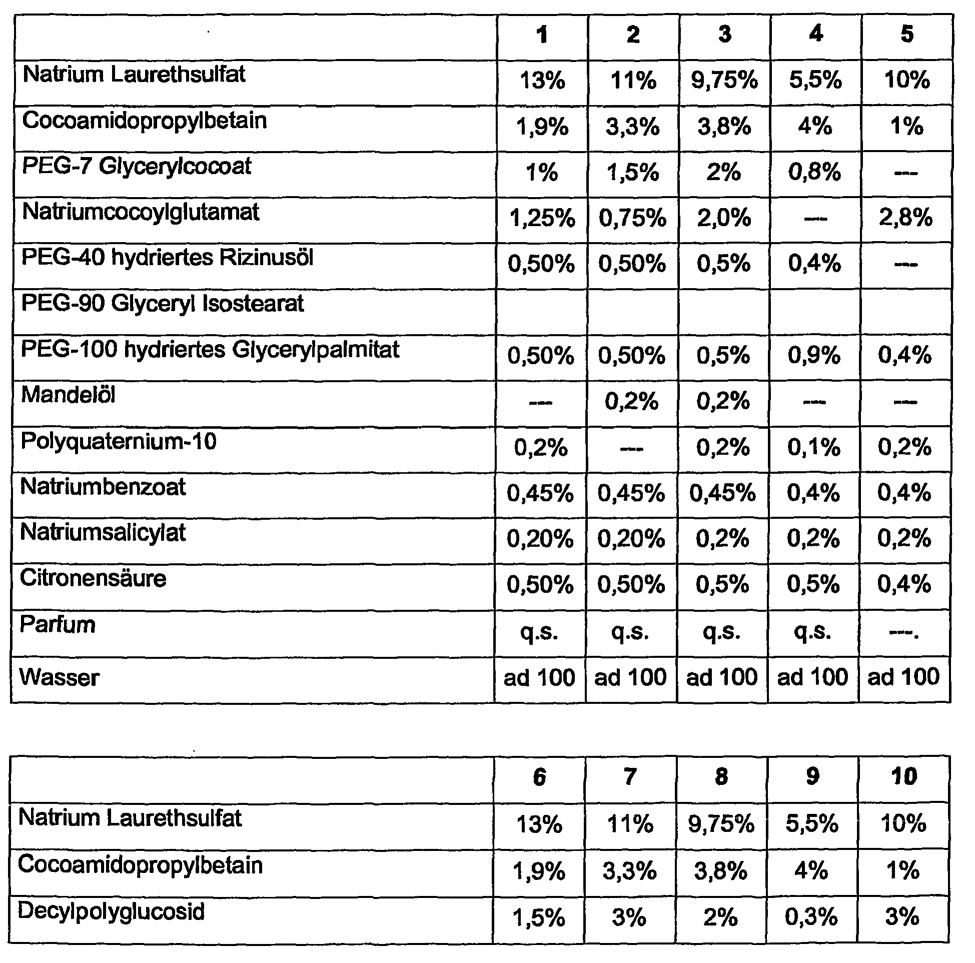

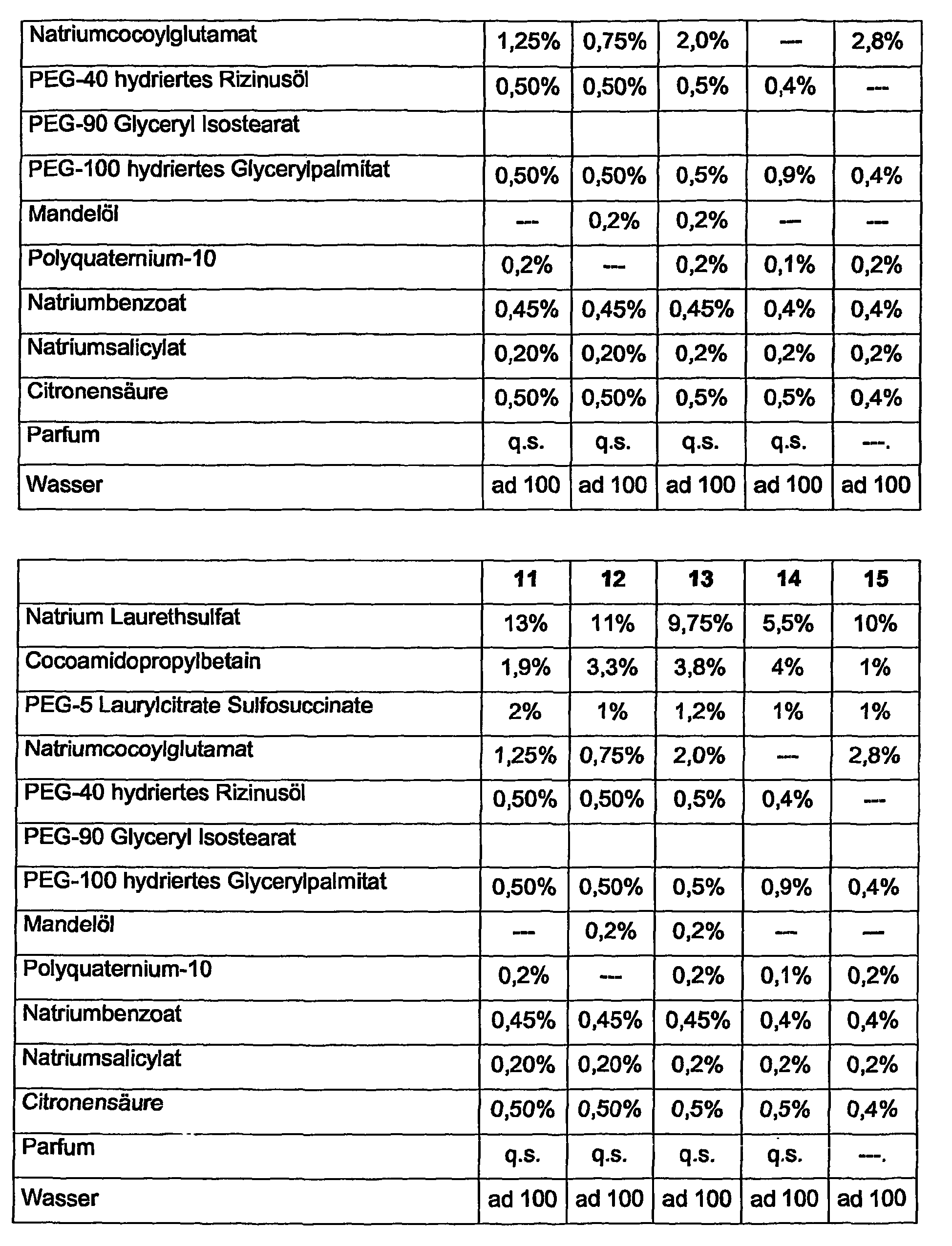

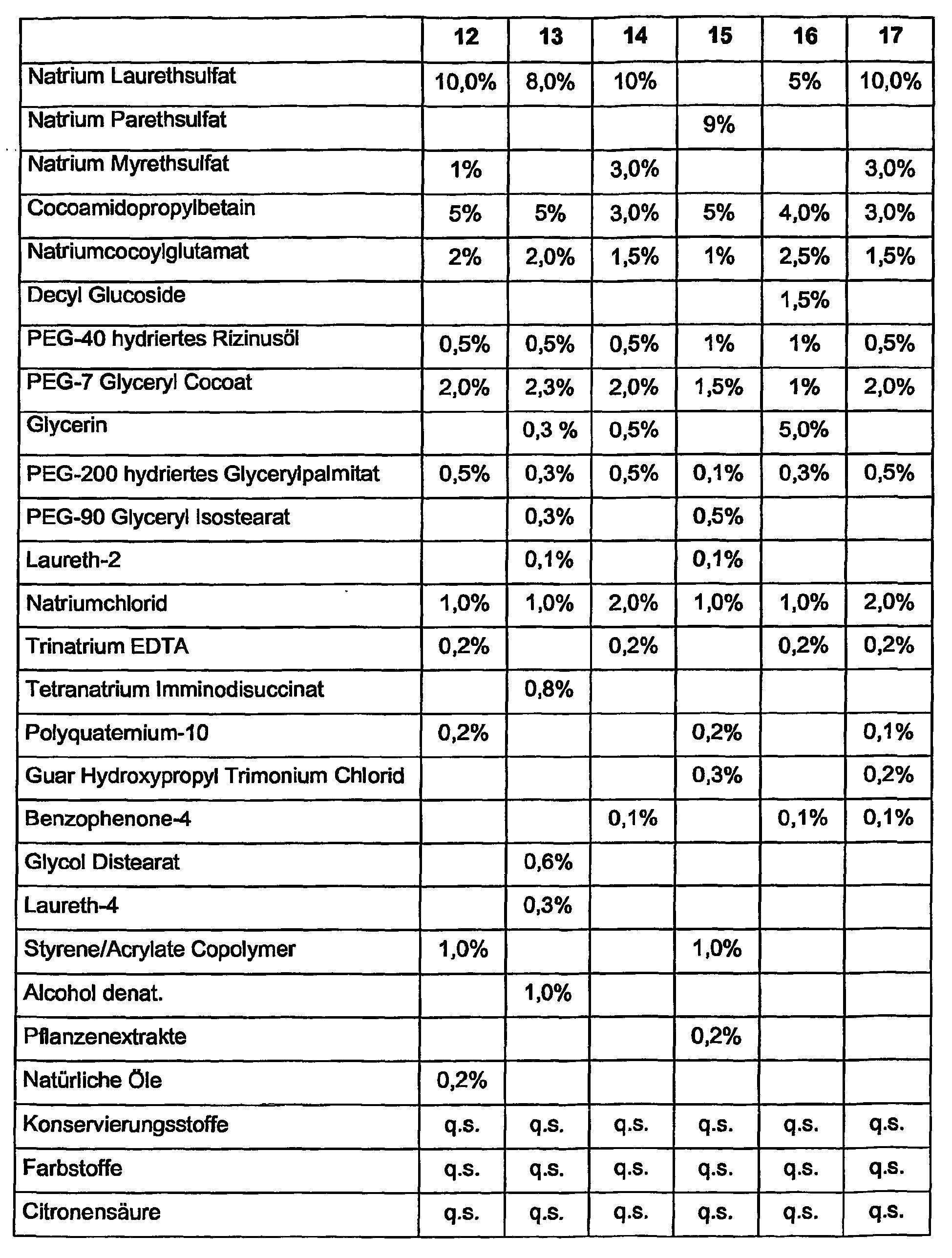

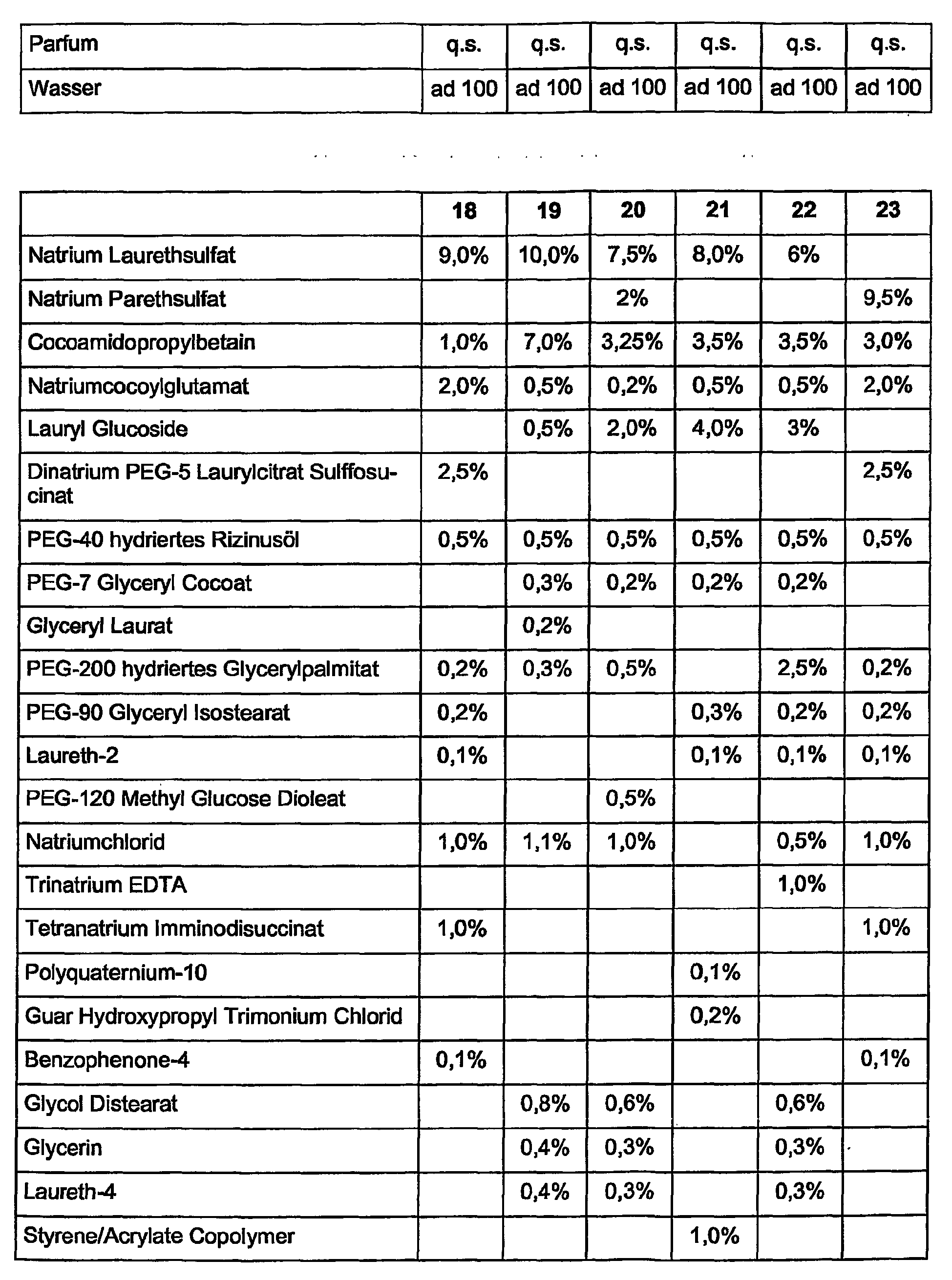

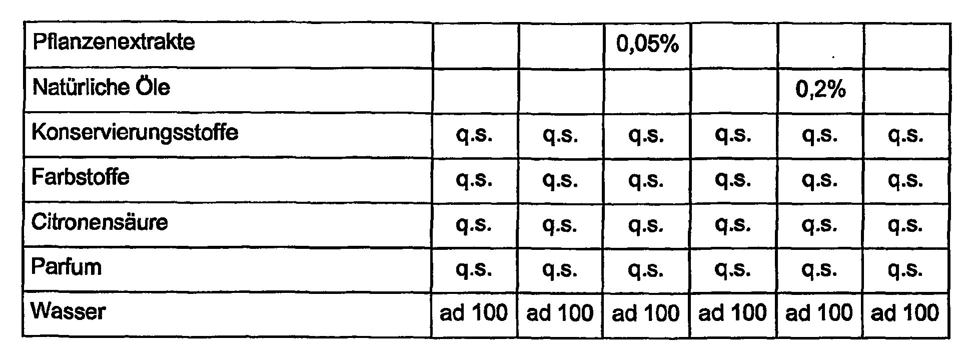

## Patentansprüche

1. Verwendung von Tensidsystemen aus
(1) Alkylethersulfat,
(2) Alkylamidopropylbetain,
(3) einem weiteren Tensid gewählt aus der Gruppe Alkylpolyglucoside, Ethoxylierte Triglyceride und Salze von Citronensäurealkylpolyglycolester-sulfosuccinaten zur Verringerung der Schädigung hauteigener Enzyme bei der Körper- oder Haarreinigung.

2. Verwendung nach Anspruch 1 **dadurch gekennzeichnet, dass** der Gehalt an weiterem Tensid (3) größer 1 Gew.%, bevorzugt größer 1,5 Gew.% ist.

3. Verwendung nach einem der vorangehenden Ansprüche **dadurch gekennzeichnet, dass** das Verhältnis von Alkylethersulfat zu weiterem Tensid (3) 10:0,5 bis 10:5, besonders bevorzugt 10:1 bis 10:3 und ganz besonders bevorzugt 10:1,8 bis 10:2,2 beträgt.

4. Verwendung nach einem der vorangehenden Ansprüche **dadurch gekennzeichnet, dass** zusätzlich Na-Cocoylglutamat enthalten ist.

5. Verwendung nach einem der vorangehenden Ansprüche **dadurch gekennzeichnet, dass** als Tensid (3) PEG-7 Glycerylcocoat, Dinatrium PEG-5 Laurylcitrat Sulfosuccinat oder Laurylglucosid gewählt wird.

## Claims

1. Use of surfactant systems of
(1) alkyl ether sulfate,
(2) alkylamidopropylbetaine,
(3) a further surfactant chosen from the group consisting of alkyl polyglucosides, ethoxylated triglycerides and salts of citric acid alkyl polyglycol ester sulfosuccinates for reducing skin-intrinsic enzyme damage when cleaning the body or hair.

2. Use according to Claim 1, **characterized in that** the content of further surfactant (3) is greater than 1 % by weight, preferably greater than 1.5% by weight.

3. Use according to one of the preceding claims, **characterized in that** the ratio of alkyl ether sulfate to further surfactant (3) is 10:0.5 to 10:5, particularly preferably 10:1 to 10:3 and very particularly preferably 10:1.8 to 10:2.2.

4. Use according to one of the preceding claims, **characterized in that** Na cocoyl glutamate is additionally present.

5. Use according to one of the preceding claims, **characterized in that** the surfactant (3) chosen is PEG-7 glyceryl cocoate, disodium PEG-5 lauryl citrate sulfosuccinate or lauryl glucoside.

## Revendications

1. Utilisation de systèmes tensioactifs, constitués par
(1) un alkyléthersulfate,
(2) une alkylamidopropylbétaïne,
(3) un autre agent tensioactif choisi dans le groupe formé par les alkylpolyglucosides, les triglycérides éthoxylés et les sels d'esters d'alkylpolyglycol et d'acide citrique-sulfosuccinates pour diminuer la dégradation d'enzymes propres à la peau lors du nettoyage du corps ou des cheveux.

2. Utilisation selon la revendication 1, **caractérisée en ce que** la teneur en autre agent tensioactif (3) est supérieure à 1% en poids, de préférence supérieure à 1,5% en poids.

3. Utilisation selon l'une quelconque des revendications précédentes, **caractérisée en ce que** le rapport d'alkyléthersulfate à autre agent tensioactif (3) est de 10:0,5 à 10:5, de manière particulièrement préférée de 10:1 à 10:3 et de manière tout particulièrement préférée de 10:1,8 à 10:2,2.

4. Utilisation selon l'une quelconque des revendications précédentes, **caractérisée en ce qu'**en outre, du cocoylglutamate de sodium est contenu.

5. Utilisation selon l'une quelconque des revendications précédentes, **caractérisée en ce qu'**on choisit, comme agent tensioactif (3) du glycérylcocoate de PEG-7, du PEG-5 laurylcitrate sulfosuccinate sodique ou du laurylglucoside.
